# EUROPEAN PATENT APPLICATION

(11) **EP 4 772 645 A1**
(43) Date of publication of application: **08.07.2026**
(21) Application number: 25810239.1
(22) Date of filing: 09.05.2025
(51) Int. Cl.: C12N 15/77, C12N 9/04, C12P 13/22, C12P 13/24

(54) **MICROORGANISM INTO WHICH EXOGENOUS NAD-DEPENDENT ISOCITRATE DEHYDROGENASE IS INTRODUCED AND METHOD FOR PRODUCING L-AMINO ACID USING SAME**

(30) Priority: 10.05.2024 KR 20240061779
(71) Applicant: CJ Cheiljedang Corporation, Seoul 04560 (KR)
(72) Inventor: KIM, Gyuree, Seoul 04560 (KR); JUNG, Moo Young, Seoul 04560 (KR); PARK, Seul-Gi, Seoul 04560 (KR); KIM, Seon Hye, Seoul 04560 (KR); LEE, Sumin, Seoul 04560 (KR)
(74) Representative: Meissner Bolte Partnerschaft mbB
(86) International application number: PCT/KR2025/006251
(87) International publication number: WO 2025/234822

(57) **Abstract**

Provided are a microorganism of the genus *Corynebacterium* having an ability to produce an L-amino acid, into which an NAD-dependent isocitrate dehydrogenase protein derived from *Streptococcus mutans* or a polynucleotide encoding the same is introduced; a method of producing an L-amino acid, the method comprising the step of culturing the microorganism in a medium; a composition for producing an L-amino acid, the composition comprising the microorganism, a culture of the microorganism, a fermentation product of the microorganism, or a combination of two or more thereof; and use of the microorganism in producing an L-amino acid.

## Description

### [Technical Field]

The present disclosure relates to a microorganism of the genus *Corynebacterium* having an ability to produce an L-amino acid, into which NAD-dependent isocitrate dehydrogenase derived from *Streptococcus mutans* or a polynucleotide encoding the same is introduced; a method of producing an L-amino acid, the method comprising the step of culturing the microorganism in a medium; a composition for producing an L-amino acid, the composition comprising the microorganism, a culture of the microorganism, a fermentation product of the microorganism, or a combination of two or more thereof; and use of the microorganism in producing an L-amino acid.

### [Background Art]

A process of producing target substances (e.g., amino acids) in microorganisms has been studied extensively as an environmentally friendly and safe production method, and among them, research has been continuously conducted on producing large quantities of target substances in microorganisms of the genus *Corynebacterium.* Microorganisms of the genus *Corynebacterium* (*Corynebacterium* sp.), particularly, *Corynebacterium glutamicum* are Gram-positive microorganisms frequently used in the production of L-amino acids and other useful substances.

L-amino acids are basic structural units of proteins and are used as an important material for pharmaceutical raw materials, food additives, animal feeds, nutritional supplements, pesticides, disinfectants, etc. For the production of L-amino acids and other useful substances, a variety of studies are being conducted to develop microorganisms with high-efficiency production and technologies for fermentation processes. For example, target substance-specific approaches are mainly used, in which the expression of a gene encoding an enzyme involved in L-tryptophan biosynthesis is increased, or in which genes unnecessary for the biosynthesis are removed (US 8945907 B2).

L-tryptophan, which is one of the essential amino acids, has been widely used as a raw material for feed additives, pharmaceutical products such as infusion solutions, etc., and health food ingredients, etc., and may be produced by a chemical synthesis method, an enzyme reaction method, a fermentation method, etc. However, the direct fermentation method using a microorganism is mostly used at present. According to previous studies, the highest level of energy is required for biosynthesis of tryptophan among 20 amino acids, which was demonstrated by practical intracellular quantitative analysis (Proc. Natl. Acad. Sci. USA(2002) V99, pp3695-3700). Accordingly, research on effectively increasing the L-tryptophan production is still needed.

L-histidine is one of the 20 standard amino acids and is classified as an essential amino acid for growing children. L-histidine is involved in important physiological processes such as antioxidation, immune regulation, etc., and thus is used in the medical industry, such as an agent for treating gastric ulcer, a raw material for a cardiovascular agent, amino acid infusion solutions, etc. Histidine is mainly found in hemoglobin, and is primarily produced by a protein hydrolysis extraction method using blood meal as a raw material. However, it has disadvantages such as low efficiency, environmental pollution, etc. On the other hand, production of L-histidine through microbial fermentation is possible, but large-scale industrialization has not yet been accomplished. Accordingly, research on effectively increasing L-histidine production ability is still needed.

### [Disclosure]

### [Technical Problem]

An object to be achieved in the present disclosure is to provide a microorganism into which a foreign NAD-dependent isocitrate dehydrogenase is introduced, and a method of producing an L-amino acid using the same.

### [Technical Solution]

One aspect of the present disclosure provides a microorganism of the genus *Corynebacterium* having an ability to produce an L-amino acid, into which an NAD-dependent isocitrate dehydrogenase protein derived from *Streptococcus mutans* or a polynucleotide encoding the same is introduced.

In one specific embodiment, the L-amino acid may be any one or more selected from the group consisting of L-tryptophan and L-histidine.

In another specific embodiment, the NAD-dependent isocitrate dehydrogenase protein derived from *Streptococcus mutans* may comprise an amino acid sequence of SEQ ID NO: 1 or an amino acid sequence having 80% or more sequence identity thereto.

In still another specific embodiment, the NAD-dependent isocitrate dehydrogenase protein derived from *Streptococcus mutans* may be encoded by *icd* gene.

In still another specific embodiment, the polynucleotide encoding the NAD-dependent isocitrate dehydrogenase protein derived from *Streptococcus mutans* may comprise a nucleotide sequence of SEQ ID NO: 2.

In still another specific embodiment, the microorganism of the genus *Corynebacterium* may be *Corynebacterium glutamicum.*

With regard to the microorganism according to any one of the aforementioned specific embodiments, the microorganism of the genus *Corynebacterium* may have an increased ability to produce an L-amino acid, as compared to an unmodified microorganism.

Another aspect of the present disclosure provides a method of producing an L-amino acid, the method comprising the step of culturing, in a medium, a microorganism of the genus *Corynebacterium* having an ability to produce an L-amino acid, into which an NAD-dependent isocitrate dehydrogenase protein derived from *Streptococcus mutans* or a polynucleotide encoding the same is introduced.

In one specific embodiment, the L-amino acid may be any one or more selected from the group consisting of L-tryptophan and L-histidine.

In another specific embodiment, the method may further comprise the step of recovering the L-amino acid from the cultured microorganism, a culture of the microorganism, a fermentation product of the microorganism, or the culture medium.

Still another aspect of the present disclosure provides a composition for producing an L-amino acid, the composition comprising a microorganism of the genus *Corynebacterium* having an ability to produce an L-tryptophan, into which an NAD-dependent isocitrate dehydrogenase protein derived from *Streptococcus mutans* or a polynucleotide encoding the same is introduced, a culture of the microorganism, a fermentation product of the microorganism, or a combination of two or more thereof.

In one specific embodiment, the L-amino acid may be any one or more selected from the group consisting of L-tryptophan and L-histidine.

### [Advantageous Effects]

A microorganism of the genus *Corynebacterium* having an ability to produce an L-amino acid of the present disclosure, into which an NAD-dependent isocitrate dehydrogenase protein derived from *Streptococcus mutans* or a polynucleotide encoding the same is introduced, is able to produce the L-amino acid with a high yield, thereby being usefully applied to industrial production of L-amino acid.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") may comprise plural referents unless stated otherwise. Unless stated otherwise, singular terms shall comprise the plural, and plural terms shall comprise the singular. As used in the specification and appended claims of the present disclosure, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. For example, the term "about" may refer to a range from -10% to +10% of a numerical value. For another example, the term "about" may refer to a range from -5% to +5% of a given numerical value, but is not limited thereto.

As used herein, descriptions such as the terms "first, second, third,,", "i), ii), iii),," or "(a), (b), (c), (d),," are used to distinguish individual components, and when the terms are used in reference to steps of a method, use, or assay, these terms are not limited to being performed continuously or sequentially; for example, there may be no time interval between these steps, or they may be performed concurrently, or may be performed sequentially, in reverse order, or randomly with several seconds, several minutes, several hours, several days, or several months apart.

As used herein, the term "consisting of" means that the total ratio of specific features, steps, ingredients or other component(s) recited after the term is 100%. The features, steps, ingredients or other component(s) that are recited after the term "consisting of" may be essential or mandatory. For example, in addition to the features, steps, ingredients or other component(s) recited after the term "consisting of", any other features, steps, ingredients or other component(s), or non-essential features, steps, ingredients or other component(s) may be excluded.

As used herein, the term "consisting essentially of" may mean that when the features, steps, ingredients or other component(s) of the object claimed herein are not substantially affected by the presence of one or more unspecified features, steps, ingredients or other component(s), the one or more unspecified features, steps, ingredients or other component(s) may be present.

As used herein, the term "comprising" means the presence of features, steps, ingredients. or other component(s) recited after the term, and does not exclude the presence of one or more additional features, steps, ingredients or other component(s). The features, steps, ingredients or other component(s) recited after the term "comprising" herein may be essential or mandatory. However, in some embodiments, the term may further comprise any other or non-essential features, steps, ingredients or other component(s).

### Protein, Polypeptide

As used herein, the term "protein" or "polypeptide" refers to a polymer or oligomer of consecutive amino acid residues. In the present disclosure, the terms "polypeptide", "protein", and "peptide" may be used interchangeably with each other.

As used herein, the term "mature polypeptide or mature protein" means a polypeptide or protein in a form without a signal sequence or propeptide sequence. A mature polypeptide or mature protein may be a functional form of a polypeptide or protein. The mature polypeptide or mature protein may refer to a polypeptide in a final form after translation; and/or post-translational modification. For example, examples of the post-translational modification may comprise N-terminal processing, C-terminal truncation, glycosylation, phosphorylation, leader sequence removal, etc., but are not limited thereto.

In the present disclosure, unless indicated otherwise, the amino acid sequences are described in an N-terminal to C-terminal direction.

With respect to amino acid sequences in the present disclosure, it is apparent that a polypeptide or protein "comprising" an amino acid sequence described by a specific sequence number, a polypeptide or protein "consisting of" an amino acid sequence described by a specific sequence number, or a polypeptide or protein "having" an amino acid sequence described by a specific sequence number may also comprise any polypeptide or protein in which some amino acid(s) is(are) deleted, modified, substituted, or added, as long as it has the identical or corresponding activity to that of the polypeptide or protein consisting of the amino acid sequence of the corresponding sequence number. For example, the polypeptide or protein may also comprise a polypeptide or protein having addition or deletion of an amino acid(s) which does/do not alter the function of the protein inside or before/after (N-terminus or C-terminus) the polypeptide or protein, a naturally occurring mutation, a silent mutation thereof, or a conservative substitution, as long as it has the identical or corresponding activity.

Further, for example, a polypeptide or protein conjugated to an N-terminal signal (or leader) sequence that is co-translationally or post-translationally involved in the protein (polypeptide) translocation, or a polypeptide or protein conjugated to another sequence or linker so as to enable identification, purification, or synthesis of the polypeptide or protein may also be comprised in the scope of the polypeptide or protein having the amino acid sequence described by the specific sequence number.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids comprise arginine, lysine, and histidine; negatively charged (acidic) amino acids comprise glutamic acid and aspartic acid; amino acids having a nonpolar side chain (nonpolar amino acids) comprise glycine, alanine, valine, leucine, isoleucine, methionine, phenylalanine, tryptophan, and proline; amino acids having a polar or hydrophilic side chain (polar amino acids) comprise serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, the 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto. Usually, conservative substitution may hardly affect or not affect the activity of polypeptides or proteins.

### Gene, Polynucleotide

As used herein, the term "gene" narrowly refers to a polynucleotide encoding a functional molecule, and broadly refers to a polynucleotide comprising the polynucleotide encoding a functional molecule and the regions upstream and downstream of the polynucleotide. In some embodiments, the functional molecule may be RNA or protein, and the gene may have a sequence (intron) inserted between each coding region (exon).

As used herein, the term "polynucleotide", "nucleic acid", or "nucleic acid molecule" refers to a DNA (e.g., cDNA or genomic DNA) or RNA (e.g., mRNA) strand of a certain length or longer as a polymer of nucleotides in which nucleotide monomers are connected in a long chain form by covalent bonds. In the present disclosure, the terms "polynucleotide", "nucleic acid", and "nucleic acid molecule" may be used interchangeably with each other.

### Identity, Homology

As used herein, the term "identity" or "homology" refers to a degree of similarity between two given amino acid or nucleotide sequences, and may be expressed as a percentage. The terms "homology" and "identity" may often be used interchangeably with each other.

The sequence homology or identity of a conserved polynucleotide or polypeptide is determined by standard alignment algorithms, and the default gap penalty established by a program to be used may be used together.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined by comparing sequence information using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later), or the GAP computer program such as the Smith-Waterman algorithm (Smith and Waterman, Adv. Appl. Math (1981) 2:482) (comprising the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073)). For example, the homology, similarity, or identity may be determined using BLAST or ClustalW of the National Center for Biotechnology Information.

Further, whether any two polynucleotide sequences have homology, similarity, or identity with each other may be identified by a Southern hybridization experiment under appropriate hybridization conditions, and the appropriate hybridization conditions may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences may generally hybridize under stringent conditions to the entirety of the sequence or at least about 50%, 60%, 70%, 80%, or 90% of the full-length sequence.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise conditions in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

The hybridization may occur between nucleotides having bases of complementary sequences, but the hybridized polynucleotides may comprise some mismatches between bases depending on the stringency of hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may comprise isolated nucleotide fragments complementary to the entire sequence as well as nucleotide sequences substantially similar thereto.

For example, polynucleotides having homology or identity to the polynucleotide of the present disclosure may be detected by hybridization at a Tm value of 55°C. Further, the Tm value may be 60°C, 63°C, or 65°C, but is not limited thereto, and may be appropriately adjusted by those skilled in the art.

The appropriate stringency for hybridizing the polynucleotides depends on the length of the polynucleotides and the degree of complementation, and these variables are well known in the art (e.g., J. Sambrook et al., supra).

### Nucleic acid construct, Vector, Transformation

As used herein, the term "nucleic acid construct" refers to an artificially designed single- or double-stranded nucleic acid molecule comprised in a vector that may be used to integrate a target genetic material into a suitable host or host cell. For example, the nucleic acid construct may comprise a transgene delivered via a transformation vector that allows an inserted sequence to be replicated and/or expressed in a host cell. For example, the transgene may be cloned from an existing sequence or artificially synthesized.

As used herein, the term "vector" means a DNA construct for delivering a target polynucleotide into a suitable host or host cell.

For example, the vector may be one comprising a nucleotide sequence of a polynucleotide encoding a target polypeptide which is operably linked to a suitable expression regulatory region (or expression control sequence) so that the target polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell (microorganism), and then replicate or function independently of the host genome, or may be integrated into the genome itself.

In addition, for example, the vector of the present disclosure may comprise a sequence for inserting a target polynucleotide into the chromosome. The insertion of the polynucleotide into the chromosome using the vector may be performed by any method known in the art, for example, by homologous recombination, but is not limited thereto.

The vector used in the present disclosure is not particularly limited, but any vector known in the art may be used. Examples of commonly used vectors may comprise natural or recombinant plasmids, cosmids, viruses, and bacteriophages. For example, pWE15, M13, MBL3, MBL4, IXII, ASHII, APII, t10, t11, Charon4A, and Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, a pDZ, pDC, pDC24, pACYC177, pACYC184, pCL, pECCG117, pUC19, pBR322, pMW118, pCC1BAC vector, etc. may be used.

The vector may further comprise a selection marker to confirm transformation into a host cell, or furthermore, insertion into the chromosome in the host cell. The selection marker is for selecting the cells transformed with vectors or for confirming whether the target polynucleotide has been inserted into the chromosome, and markers that confer selectable phenotypes such as drug resistance, auxotrophy, resistance to cytotoxic agents, or expression of surface polypeptides may be used. Only cells expressing the selection marker are able to survive or show different phenotypes under the environment treated with the selective agent, and thus the transformed cells may be selected.

As used herein, the term "transformation" means that a target polynucleotide or a vector comprising the same is introduced into a host cell (microorganism) so that the genetic traits of the host cell (microorganism) are changed. The transformed polynucleotide may be located either by being inserted into the chromosome of the host cell (microorganism) or by being located outside the chromosome. Further, the polynucleotide comprises DNA or RNA. The polynucleotide may be introduced in an appropriate form depending on the purpose of introduction. For example, the polynucleotide for expressing the target polypeptide may be introduced into a host cell (microorganism) in the form of an expression cassette, which is a gene construct comprising all elements required for self-expression. The expression cassette may usually comprise a promoter operably linked to a coding sequence of a target polypeptide, a transcription termination signal, a ribosome binding site, and a translation termination signal. The expression cassette may be in the form of a self-replicable expression vector. Additionally, the polynucleotide may be introduced into a host cell (microorganism) as it is and operably linked to sequences required for expression in the host cell (microorganism), but is not limited thereto.

As used herein, the term "operably linked" refers to a configuration of placing a regulatory sequence in an appropriate position to direct expression of a coding sequence. Thus, the term "operably linked" comprises an attachment or linking between a regulatory region of a functional domain having a known or desired activity such as a promoter, a terminator, a signal sequence, or an enhancer, and a target (gene or polypeptide) such that the expression, secretion, or function of the target (gene or polypeptide) may be regulated in accordance with the known or desired activity. For example, the term means that the polynucleotide sequence is functionally linked to a promoter sequence that initiates and mediates transcription of the polynucleotide encoding the polypeptide.

As used herein, the term "expression" comprises any step involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule comprising a target polynucleotide sequence and an operably linked regulatory sequence for expression thereof. For example, the expression vector may comprise a nucleotide sequence of a polynucleotide encoding a target polypeptide which is operably linked to a suitable expression regulatory region (or expression control sequence) so that the target polypeptide may be expressed in a suitable host.

As used herein, the term "regulatory sequence" refers to a polynucleotide sequence required for the regulation of expression of a target polynucleotide sequence. Each regulatory sequence may be native (derived from the same origin) or foreign (derived from a different gene) to the coding sequence, or a mutant sequence thereof or other artificial sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

As used herein, the term "genetic recombination" refers to a natural or artificial process in which the elements constituting genes, such as DNA or RNA, are altered from their original sequence during disassembly and reassembly.

As used herein, the term "recombinant gene" refers to a gene with a new genomic composition that develops as a result of genetic recombination, e.g., chemical synthesis or genetic engineering technology, etc. As used herein, the terms "recombinant gene", "recombinant DNA" and "recombinant polynucleotide" may be used interchangeably with each other. For example, the recombinant gene may comprise an artificial combination of nucleic acid fragments, such as regulatory sequences, that are not found in nature.

As used herein, the term "recombinant protein" refers to a protein produced as a result of genetic recombination.

### Microorganism

As used herein, the term "microorganism (or strain)" comprises all wild-type microorganisms, or prokaryotic eukaryotic microorganisms in which genetic modification naturally or artificially occurs, and it may be a microorganism in which a specific mechanism is weakened or enhanced due to insertion of a foreign gene or an activity enhancement or inactivation of an endogenous gene, and it may be a microorganism comprising genetic modification for the production of a desired polypeptide, protein, or product. As used herein, the terms "microorganism", "strain", "host", and "host cell" may be used interchangeably with each other.

As used herein, the term "recombinant microorganism" refers to a microorganism that has been genetically modified and exhibits a different genotype and/or phenotype, compared to a naturally occurring microorganism (e.g., when the genetic modifications have an effect on a coding nucleic acid sequence of a microorganism), and may comprise progeny or all potential progeny of the microorganism. As used herein, the terms "recombinant microorganism", "genetically modified microorganism", "recombinant host cell", "recombinant cell" and "recombinant strain" may be used interchangeably with each other. The recombinant microorganism, for example, may express genes that are not found in the native (non-recombinant) form; or may not express genes that are expressed in its native form, or may express native genes in a manner different from that expressed in its native form.

For example, the microorganism of the present disclosure may be a microorganism (e.g., recombinant microorganism) into which the NAD-dependent isocitrate dehydrogenase protein or the polynucleotide encoding the same is introduced, but is not limited thereto.

As used herein, the term "microorganism having an ability to produce an L-amino acid", which is a microorganism capable of producing the L-amino acid in an organism, may comprise any microorganism prepared by providing the ability to produce the L-amino acid to a microorganism inherently not having the ability to produce the L-amino acid, or a microorganism that inherently has the ability to produce the L-amino acid. The ability to produce the L-amino acid may be conferred or enhanced by species improvement.

As used herein, the term "unmodified microorganism (strain)" does not exclude microorganisms (strains) comprising a mutation that may occur naturally, and may be a wild-type microorganism (strain) or a natural microorganism (strain) itself or may be a microorganism (strain) before the trait is changed by genetic variation due to natural or artificial factors. In the present disclosure, the term "unmodified microorganism (strain)" may be used interchangeably with "microorganism (strain) before being modified", "unvaried microorganism (strain)", "parent microorganism", "parent strain", "wild-type microorganism (strain)", "reference microorganism (strain)" or "standard microorganism (strain)". In the present disclosure, the unmodified microorganism may refer to a microorganism (strain), into which the NAD-dependent isocitrate dehydrogenase protein of the present disclosure or the polynucleotide encoding the same is not introduced, or a microorganism (strain) before introduction thereof, but is not limited thereto. Further, the unmodified microorganism in the present disclosure may be a microorganism not comprising a polypeptide consisting of SEQ ID NO: 1 or a polynucleotide consisting of SEQ ID NO: 2, but is not limited thereto.

### Increase of Protein (Polypeptide) Activity

As used herein, the term "increase" of protein (polypeptide) activity means that the activity of a protein (polypeptide) in a host cell (microorganism) is enhanced, as compared to its endogenous activity. The increase may be used interchangeably with the terms such as activation, up-regulation, overexpression, enhancement, etc. The host cell (microorganism) may be a prokaryotic or eukaryotic microorganism.

The increase of protein (polypeptide) activity may comprise both cases where a protein (polypeptide) activity not endogenously possessed by a host cell (microorganism) is exhibited, or where enhanced protein (polypeptide) activity is exhibited, as compared to its endogenous activity or the activity before modification.

For example, "the protein (polypeptide) activity not endogenously possessed is exhibited" or "the enhanced protein (polypeptide) activity is exhibited" may be caused by "introduction of a polypeptide (protein)", but is not limited thereto.

As used herein, the term "introduction" of protein (polypeptide) means that a gene not originally possessed by a microorganism is expressed within the microorganism to exhibit the activity of a specific protein or to exhibit the enhanced, increased, or improved polypeptide activity, as compared to endogenous activity of the corresponding protein or the activity before modification. For example, a polynucleotide encoding a specific protein (polypeptide) may be introduced into the chromosome in a host cell (microorganism), or a vector comprising the polynucleotide encoding the specific protein (polypeptide) may be introduced into the host cell (microorganism), thereby exhibiting its activity.

The "endogenous activity" means the activity of a specific protein (polypeptide) originally possessed by a host cell (microorganism) before the trait is changed or by an unmodified host cell (microorganism), when the trait is changed by genetic variation due to natural or artificial factors. This may be used interchangeably with "activity before modification".

The increase in the activity of a protein (polypeptide) as compared to the endogenous activity means that the activity and/or concentration (expression level) of the protein (polypeptide) of a host cell (microorganism) is improved, as compared to the activity and/or concentration (expression level) of the protein (polypeptide) originally possessed by a host cell (microorganism) before transformation or an unmodified host cell (microorganism).

For example, the increase indicates that the activity of the corresponding protein (polypeptide) originally absent appears, or its activity or concentration is generally increased to about 1% or more, about 10% or more, about 25% or more, about 50% or more, about 75% or more, about 100% or more, about 150% or more, about 200% or more, about 300% or more, about 400% or more, or about 500% or more, up to about 1000% or about 2000% or more, based on the activity or concentration of a host cell (microorganism) before transformation or an unmodified host cell (microorganism), but is not limited thereto.

The increase of the activity of the protein (polypeptide) may be achieved through the introduction of a foreign protein (polypeptide) or the increase of the activity of the protein (polypeptide). The increase of the activity of the protein (polypeptide) may be confirmed by an increase in the degree of activity and the expression level of the corresponding protein (polypeptide), or an increase in the amount of a product due to the activity of the corresponding protein (polypeptide).

The increase of the activity of the protein (polypeptide) may be achieved by various methods well known in the art, and the method is not limited as long as the activity of the target protein (polypeptide) may be increased, as compared to that of the host cell (microorganism) before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the protein (polypeptide) of the present disclosure may be:
1) increasing the intracellular copy number of a polynucleotide encoding the protein (polypeptide);
2) modifying the expression regulatory region of a gene encoding the protein (polypeptide) on the chromosome (e.g., introducing a modification into the expression regulatory region, replacing it with a sequence having a stronger activity, or inserting a sequence having a stronger activity);
3) modifying the nucleotide sequence encoding the initiation codon or 5'-UTR of the gene transcript encoding the protein (polypeptide);
4) modifying the amino acid sequence of the protein (polypeptide) such that the activity of the protein (polypeptide) is enhanced;
5) modifying the polynucleotide sequence encoding the protein (polypeptide) such that the activity of the protein (polypeptide) is enhanced (e.g., modifying the polynucleotide sequence of the protein (polypeptide)-encoding gene to encode a protein (polypeptide) that has been modified to enhance the activity of the protein (polypeptide));
6) introducing a foreign protein (polypeptide) exhibiting the activity of the protein (polypeptide) or a foreign polynucleotide encoding the same;
7) codon-optimization of a polynucleotide encoding the protein (polypeptide);
8) analyzing the tertiary structure of the protein (polypeptide) thereby selecting and modifying the exposed site, or chemically modifying the same; or
9) controlling the intracellular localization of the protein (polypeptide); or
10) a combination of two or more selected from 1) to 9), but is not particularly limited thereto.

For example,
1) the increasing the intracellular copy number of a polynucleotide encoding the protein (polypeptide) may be achieved by introducing a vector comprising a polynucleotide encoding the protein (polypeptide), which is operably linked to a suitable regulatory sequence, into a host cell (microorganism). Alternatively, it may be achieved by introducing one copy or two or more copies of polynucleotides encoding the protein (polypeptide), which are operably linked to a suitable regulatory sequence, into the chromosome of the host cell (microorganism). The introduction into the chromosome may be performed by introducing a vector, which is able to insert the polynucleotide into the chromosome of the host cell (microorganism), into the host cell (microorganism), but is not limited thereto. The vector is as described above. The regulatory sequence may be native (derived from the same origin) or foreign (derived from different genes) to the coding polynucleotide sequence, or may be a mutant sequence thereof or other artificial sequence, and may induce the expression of the polynucleotide in the host cell (microorganism).
2) The replacing the expression regulatory region (or expression regulatory sequence) of a gene encoding the protein (polypeptide) on the chromosome with a sequence having a strong activity may be achieved, for example, by introducing a modification into the sequence through deletion, insertion, substitution, or a combination thereof to further enhance the activity of the expression regulatory region, or by replacing the sequence with a sequence having stronger activity. The expression regulatory region may comprise, but is not particularly limited to, a promoter, an operator sequence, a sequence encoding a ribosome-binding site, and a sequence regulating the termination of transcription and translation, etc. For example, it may be replacing the original promoter with a strong promoter, but is not limited thereto.
   Examples of known strong promoters may comprise cj1 to cj7 promoters (US 7662943 B2), lac promoter, trp promoter, trc promoter, tac promoter, lambda phage PR promoter, PL promoter, tet promoter, gapA promoter, SPL7 promoter, SPL13(sm3) promoter (US 10584338 B2), O2 promoter (US 10273491 B2), tkt promoter, and yccA promoter, etc., but are not limited thereto.
3) The modifying the nucleotide sequence encoding the initiation codon or 5'-UTR of the gene encoding the protein (polypeptide) may be achieved, for example, by modifying the nucleotide sequence to encode another initiation codon having a higher expression rate of the protein (polypeptide) compared to the endogenous initiation codon, or a RBS (ribosome binding site) sequence having a higher expression rate of the protein (polypeptide) compared to the endogenous RBS sequence, but is not limited thereto.
4) and 5) The modifying the amino acid sequence or the polynucleotide sequence of the protein (polypeptide) may be achieved by introducing a modification into the sequence through deletion, insertion, or substitution of the amino acid sequence of the protein (polypeptide) or the polynucleotide sequence encoding the protein (polypeptide), or a combination thereof to enhance the activity of the protein (polypeptide), or by replacing it with an amino acid sequence or a polynucleotide sequence modified to increase the activity, but is not limited thereto. The replacement may be, for example, performed by inserting the polynucleotide into the chromosome by homologous recombination, but is not limited thereto.
6) The introducing a foreign polynucleotide exhibiting the activity of the protein (polypeptide) may be achieved by introducing into a host cell (microorganism) a foreign polynucleotide encoding a protein (polypeptide) that exhibits identical/similar activity to that of the protein (polypeptide). The foreign polynucleotide may be used without limitation regardless of its origin or sequence as long as it exhibits identical/similar activity to that of the protein (polypeptide). The method employed in the introduction may be performed by those of ordinary skill in the art by appropriately selecting a known transformation method, and the expression of the introduced polynucleotide in the host cell enables the production of the protein (polypeptide), thereby increasing its activity.
7) The codon-optimization of the polynucleotide encoding the protein (polypeptide) may be achieved by codon-optimization of an endogenous polynucleotide to increase the transcription or translation within a host cell (microorganism), or by optimizing the codons thereof such that optimized transcription and translation of the foreign polynucleotide may be achieved within the host cell (microorganism).
8) The analyzing the tertiary structure of the protein (polypeptide), thereby selecting and modifying the exposed site, or chemically modifying the same may be achieved, for example, by comparing the sequence information of the protein (polypeptide)to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.
9) The controlling the intracellular localization of the protein (polypeptide) may mean targeting the protein (polypeptide) to a specific organelle within cells or to a specific intracellular space, for example, targeting to the periplasm or cytoplasm through the addition or removal of a leader sequence that functions in targeting the protein (polypeptide), but is not limited thereto.

Such an increase of the protein (polypeptide) activity may mean that the activity or concentration of the corresponding protein (polypeptide) is increased relative to the activity or concentration of a protein (polypeptide) expressed in a wild-type or a host cell (microorganism) before modification, or that the amount of products produced from the corresponding protein (polypeptide) is increased, but is not limited thereto.

The modification of a part or all of the polynucleotide in the host cell (microorganism) of the present disclosure may be achieved by (a) homologous recombination through a vector for chromosomal insertion or genome editing using an engineered nuclease (e.g., CRISPR-Cas9), and/or (b) induction by light, such as ultraviolet rays and radiation, etc. and/or chemical treatments, but is not limited thereto.

### Culturing

As used herein, the term "culturing" means that microorganisms are grown under appropriately controlled environmental conditions. The culturing process may be performed in a suitable culture medium and under culture conditions known in the art. Such a culturing process may be easily adjusted for use by those skilled in the art according to the microorganism selected. Specifically, the culturing may be a batch culture, a continuous culture, and/or a fed-batch culture, but is not limited thereto.

As used herein, the term "medium" refers to a mixture of materials which comprises nutrient materials required for culturing microorganisms as a main ingredient, and it supplies nutrient materials and growth factors, along with water that is essential for survival and growth. Specifically, the medium and other culture conditions used for culturing the microorganism of the present disclosure may be any medium used for common culture of microorganisms without any particular limitation. For example, the microorganism of the present disclosure may be cultured under aerobic conditions in a common medium comprising an appropriate carbon source, nitrogen source, phosphorus source, inorganic compounds, amino acids, and/or vitamins, etc., while adjusting temperature, pH, etc. For example, the culture medium for microorganisms of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. In addition, natural organic nutrients such as starch hydrolysate, molasses, blackstrap molasses, rice bran, cassava, sugar cane molasses, and corn steep liquor, etc. may be used. Specifically, carbohydrates such as glucose and sterilized pretreated molasses (i.e., molasses converted to reducing sugar) may be used, and in addition, various other carbon sources in an appropriate amount may be used without limitation. These carbon sources may be used alone or in combination of two or more thereof, but are not limited thereto.

The nitrogen source may comprise inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in combination of two or more thereof, but are not limited thereto.

The phosphorus source may comprise monopotassium phosphate, dipotassium phosphate, or corresponding sodium-comprising salts, etc. The inorganic compounds may comprise sodium chloride, calcium chloride, iron chloride, magnesium sulfate, iron sulfate, manganese sulfate, calcium carbonate, etc. In addition, amino acids, vitamins, and/or appropriate precursors, etc., may be comprised. These components or precursors may be added to a medium in a batch or continuous manner, but are not limited thereto.

Further, the pH of the medium may be adjusted by adding a compound such as ammonium hydroxide, potassium hydroxide, ammonia, phosphoric acid, sulfuric acid, etc. during the culturing of the microorganism of the present disclosure in an appropriate manner. In addition, bubble formation may be prevented during culturing using an antifoaming agent such as fatty acid polyglycol ester. Further, oxygen gas or a gas comprising oxygen may be injected into the medium in order to maintain aerobic conditions of the medium; or nitrogen gas, hydrogen gas, or carbon dioxide may be injected to maintain anaerobic or microaerobic conditions, without the injection of gas, but is not limited thereto.

The culturing temperature during the culturing of the present disclosure may be maintained at 20°C to 45°C, specifically at 25°C to 40°C, and the culturing may be continued for about 10 hours to about 160 hours, but is not limited thereto.

As used herein, the term "culture product" means a culture solution, a concentrated culture solution, a dry product of a culture solution, a culture filtrate, a concentrated culture filtrate, or a dry product of a culture filtrate obtained by culturing a specific microorganism in a culture medium, and means that the culture solution may comprise the specific microorganism while the culture filtrate does not substantially comprise the specific microorganism (here, it substantially means excluding a specific microorganism isolated by filtration, etc., but does not mean that the microorganism is completely excluded from the filtrate). The formulation of the culture product is not limited, and may be, for example, a liquid, emulsion, or solid.

As used herein, the term "fermentation" means a process whereby microorganisms break down organic materials using their own enzymes, excluding putrefaction. Fermentation and putrefaction proceed by similar processes. However, when useful substances are produced as a result of decomposition, it is called fermentation, and when bad smells or harmful substances are produced, it is called putrefaction.

In the present disclosure, the method of obtaining a fermented product from the microorganism is not particularly limited, and may be performed according to a method commonly used in the art or similar fields.

As used herein, the term "fermented product" may comprise not only the fermented material itself, but also all kinds of materials comprising fermented products produced from the microorganism, such as a material comprising the fermented microorganism, a fermented product produced from the fermented microorganism, a fermented product of a culture product, a concentrated fermented product; a dry product of the fermented product, a filtrate of the fermented product, a filtrate of the concentrated fermented product, a dry product of the filtrate of the fermented product, an extract of the fermented product, or a diluted solution of the fermented product, etc.

### Detailed Description of the Present Disclosure

Hereinafter, specific embodiments of the present disclosure will be described in more detail as follows.

One aspect of the present disclosure provides a microorganism of the genus *Corynebacterium* having an ability to produce an L-amino acid, into which an NAD-dependent isocitrate dehydrogenase protein derived from *Streptococcus mutans* or a polynucleotide encoding the same is introduced.

As used herein, the term "NAD-dependent isocitrate dehydrogenase (ICD)" refers to an NAD-dependent enzyme among isocitrate dehydrogenases, which catalyzes oxidative decarboxylation to produce alpha-ketoglutarate and CO₂. The NAD-dependent isocitrate dehydrogenase of the present disclosure may be used interchangeably with ICD.

Specifically, the NAD-dependent isocitrate dehydrogenase protein of the present disclosure may be a protein having the activity of NAD-dependent isocitrate dehydrogenase encoded by the *icd* gene, but the type thereof is not particularly limited as long as the protein has activity corresponding to the activity of NAD-dependent isocitrate dehydrogenase. The NAD-dependent isocitrate dehydrogenase protein encoded by the *icd* gene is known in the art, and the amino acid and polynucleotide sequences of the NAD-dependent isocitrate dehydrogenase protein may be obtained from a known database, for example, NCBI's GenBank, etc., but are not limited thereto.

For example, the NAD-dependent isocitrate dehydrogenase protein derived from *Streptococcus mutans* may comprise the amino acid sequence of SEQ ID NO: 1, or an amino acid sequence having 60% or more homology or identity thereto, but is not limited thereto as long as it has an activity of NAD-dependent isocitrate dehydrogenase. Specifically, any protein having the amino acid sequence of SEQ ID NO: 1, in which part of the sequence is deleted, modified, substituted, or added, may be comprised in the NAD-dependent isocitrate dehydrogenase protein as long as it exhibits efficacy corresponding to that of the NAD-dependent isocitrate dehydrogenase protein. Further, any protein having or comprising an amino acid sequence having at least 60%, 70%, 80%, 85%, 90%, 95%, 96%, 97%, 98%, or 99% or more homology or identity to the amino acid sequence of SEQ ID NO: 1, consisting of the amino acid sequence, or essentially consisting of the amino acid sequence, and exhibiting efficacy corresponding to that of the NAD-dependent isocitrate dehydrogenase may be comprised in the NAD-dependent isocitrate dehydrogenase protein.

Further, the sequences of the polynucleotide encoding the NAD-dependent isocitrate dehydrogenase protein derived from *Streptococcus mutans* having the amino acid sequence of SEQ ID NO: 1 or an amino acid sequence having 60% or more homology or identity thereto may be obtained, for example, based on codon information known in the art. For example, the NAD-dependent isocitrate dehydrogenase protein may be encoded by a polynucleotide having or comprising the sequence of SEQ ID NO: 2, or a nucleotide sequence having 60% or more, 70% or more, 75% or more, 80% or more, 85% or more, 90% or more, 95% or more, 96% or more, 97% or more, 98% or more, or 99% or more homology or identity to the sequence of SEQ ID NO: 2, consisting of the nucleotide sequence, or essentially consisting of the nucleotide sequence, but is not limited thereto. Further, the nucleotide sequence of SEQ ID NO: 2 may be obtained from a known database, for example, NCBI's GenBank, etc., but is not limited thereto.

In the present disclosure, the polynucleotide (gene) comprising the nucleotide sequence of SEQ ID NO: 2 may be used interchangeably with the polynucleotide (gene) having the nucleotide sequence of SEQ ID NO: 2, the polynucleotide (gene) consisting of the nucleotide sequence of SEQ ID NO: 2, or *icd* gene.

The polynucleotide of the present disclosure may undergo various modifications in the coding region without changing the amino acid sequence of the NAD-dependent isocitrate dehydrogenase protein of the present disclosure, due to codon degeneracy or in consideration of the codons preferred in an organism in which the NAD-dependent isocitrate dehydrogenase protein of the present disclosure is to be expressed. Therefore, based on codon degeneracy, it is apparent that polynucleotides which may be translated into polypeptides consisting of the amino acid sequence of the NAD-dependent isocitrate dehydrogenase protein of the present disclosure or polypeptides having homology or identity thereto may also be comprised in the polynucleotide of the present disclosure. For example, the polynucleotide of the present disclosure may be SEQ ID NO: 2, or a degenerate sequence thereof.

For another example, the polynucleotide of the present disclosure may have or comprise a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, or may consist of or essentially consist of a nucleotide sequence having 60% or more, 70% or more, 75% or more, 80% or more, 85% or more, 90% or more, 95% or more, 96% or more, 97% or more, 98% or more, or 99% or more homology or identity to SEQ ID NO: 2, but is not limited thereto.

Further, the polynucleotide of the present disclosure may comprise a probe that may be prepared from a known gene sequence, for example, any sequence which may hybridize with a sequence complementary to all or part of the polynucleotide sequence of the present disclosure under stringent conditions to encode the NAD-dependent isocitrate dehydrogenase protein of the present disclosure without limitation.

The microorganism of the genus *Corynebacterium* of the present disclosure has an ability to produce an L-amino acid.

In the present disclosure, the L-amino acid may be specifically L-tryptophan or L-histidine.

With respect to the objects of the present disclosure, the microorganism of the present disclosure may comprise all microorganisms capable of producing the desired L-amino acid, into which the NAD-dependent isocitrate dehydrogenase protein or a polynucleotide encoding the same is introduced. For example, the microorganism of the present disclosure may be a genetically modified microorganism or a recombinant microorganism, into which the NAD-dependent isocitrate dehydrogenase protein or the polynucleotide encoding the same is introduced, thereby increasing the ability to produce an L-amino acid. Specifically, the recombinant microorganism having an increased ability to produce an L-amino acid may be a microorganism having an increased ability to produce an L-amino acid, as compared to a natural wild-type microorganism or an unmodified microorganism having an endogenous activity of NAD-dependent isocitrate dehydrogenase or having no endogenous activity of NAD-dependent isocitrate dehydrogenase, but is not limited thereto.

For example, the microorganism having an ability to produce an L-amino acid, which is a prokaryotic or eukaryotic microorganism capable of producing an L-amino acid in an organism, may comprise all microorganisms that endogenously have an ability to produce an L-amino acid, or microorganisms, in which an ability to produce an L-amino acid has been imparted to a strain having no ability to produce an L-amino acid by the activity of the introduced NAD-dependent isocitrate dehydrogenase protein of the present disclosure. The ability to produce an L-amino acid may be imparted or enhanced by improvement of species.

The microorganism of the present disclosure may comprise all microorganisms into which the NAD-dependent isocitrate dehydrogenase protein or the polynucleotide encoding the same is introduced by various known methods.

For example, the recombinant microorganism having an ability to produce an L-amino acid of the present disclosure may be a microorganism, into which a foreign gene encoding the NAD-dependent isocitrate dehydrogenase protein of the present disclosure, specifically, a foreign gene encoding the NAD-dependent isocitrate dehydrogenase protein derived from *Streptococcus mutans* is introduced by transformation through a vector, thereby producing the L-amino acid.

For example, the microorganism producing an L-amino acid may be a microorganism into which a polynucleotide sequence encoding a protein comprising the amino acid sequence of SEQ ID NO: 1, or a protein comprising an amino acid sequence having at least 60%, 70%, 75%, 80%, 85%, 90%, 95%, 96%, 97%, 98%, 99%, 99.5%, 99.7% or 99.9% or more homology or identity to the amino acid sequence of SEQ ID NO: 1 is introduced.

For example, the microorganism producing an L-amino acid may be a microorganism into which a polynucleotide capable of encoding a protein comprising an amino acid sequence having at least 80% homology to the amino acid sequence of SEQ ID NO: 1; or a polynucleotide comprising the nucleotide sequence of SEQ ID NO: 2 or a nucleotide sequence having 60% or more, 70% or more, 75% or more, 80% or more, 85% or more, 90% or more, 95% or more, 96% or more, 97% or more, 98% or more, or 99% or more homology or identity to the nucleotide sequence of SEQ ID NO: 2 is introduced.

For example, the microorganism having an increased ability to produce an L-amino acid of the present disclosure may be a microorganism having an increased ability to produce an L-amino acid, as compared to an unmodified microorganism, but is not limited thereto. For example, the unmodified microorganism that is the target strain for comparing the increase in the ability to produce an L-amino acid may be CM05-9157 or CA14-0114 strain, but is not limited thereto.

For example, the microorganism having an increased ability to produce an L-amino acid may have an increased ability to produce an L-amino acid by about 1% or more, specifically, about 1% or more, about 2.5% or more, about 5% or more, about 6% or more, about 7% or more, about 8% or more, about 9% or more, about 10% or more, about 15% or more, about 20% or more, about 25% or more, or about 26% or more (the upper limit is not particularly limited, for example, about 200% or less, about 150% or less, about 100% or less, about 50% or less, about 45% or less, about 40% or less, or about 35% or less), as compared to the ability to produce an L-amino acid of a parent microorganism (parent strain) before modification or an unmodified microorganism, but is not limited thereto, as long as it has an increased + value compared to the production ability of the parent microorganism (parent strain) before modification or the unmodified microorganism. For another example, the recombinant strain having an increased ability to produce an L-amino acid may have an increased ability to produce an L-amino acid by about 1.1 times or more, about 1.15 times or more, about 1.2 times or more, about 1.25 times or more, or about 1.26 times or more (the upper limit is not particularly limited, for example, about 10 times or less, about 5 times or less, about 3 times or less, about 2 times or less, about 1.5 times or less, about 1.4 times or less, about 1.35 times or less), as compared to that of the parent microorganism (parent strain) before modification or the unmodified microorganism, but is not limited thereto.

For example, the microorganism having an ability to produce an L-amino acid of the present disclosure may be a prokaryotic cell or eukaryotic cell, but may specifically be a prokaryotic cell. The prokaryotic cell may comprise, for example, a microorganism belonging to the genus *Escherichia* (*Escherichia* sp.), the genus *Erwinia* (*Erwinia* sp.), the genus *Serratia* (*Serratia* sp.), the genus *Providencia* (*Providencia* sp.), the genus *Corynebacterium* (*Corynebacterium* sp.), the genus *Pseudomonas* (*Pseudomonas* sp.), the genus *Leptospira* (*Leptospira* sp.), the genus *Salmonella* (*Salmonella* sp.), the genus *Brevibacteria* (*Brevibacteria* sp.), the genus *Hypomononas* (*Hypomononas* sp.), the genus *Chromobacterium* (*Chromobacterium* sp.), and the genus *Norcardia* (*Norcardia* sp.), but is not limited thereto. Specifically, the microorganism may be a microorganism of the genus *Escherichia* or *Corynebacterium.* More specifically, the microorganism may be a microorganism of the genus *Corynebacterium.*

With regard to the microorganism according to any one of the aforementioned specific embodiments, the microorganism of the present disclosure may be a microorganism of the genus *Corynebacterium.*

The microorganism of the genus *Corynebacterium* may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli*, *Corynebacterium imitans*, *Corynebacterium testudinoris*, or *Corynebacterium flavescens.* More specifically, the microorganism of the present disclosure may be *Corynebacterium glutamicum,* but is not limited thereto.

Meanwhile, the microorganism of the genus *Corynebacterium* having an ability to produce an L-amino acid of the present disclosure may comprise all of a natural wild-type microorganism itself, a microorganism of the genus *Corynebacterium* in which the activity of a gene associated with the mechanism of L-amino acid production is increased or decreased, thereby having an enhanced ability to produce an L-amino acid, or a microorganism of the genus *Corynebacterium* in which the activity of a foreign gene is introduced or increased, thereby having an enhanced ability to produce an L-amino acid.

Another aspect of the present disclosure provides a method of producing an L-amino acid, the method comprising the step of culturing, in a medium, a microorganism of the genus *Corynebacterium* having an ability to produce an L-amino acid, into which the NAD-dependent isocitrate dehydrogenase protein derived from *Streptococcus mutans* of the present disclosure or the polynucleotide encoding the same is introduced.

In the method of the present disclosure, the culturing of the microorganism may be performed using any culture conditions and culture methods known in the art. Such a culturing process may be easily adjusted for use by those skilled in the art according to the microorganism selected.

The L-amino acid produced by the culturing of the present disclosure may be released into the medium or remain in the cells.

In a specific embodiment, the method of producing an L-amino acid of the present disclosure may further comprise a step of preparing the microorganism of the present disclosure, a step of preparing a medium for culturing the strain, or a combination thereof (regardless of the order, in any order), for example, prior to the culturing step.

The method of producing an L-amino acid of the present disclosure may further comprise a step of recovering the desired substance, specifically the L-amino acid, from the cultured microorganism, a culture product of the microorganism, a fermented product of the microorganism, or the culture medium. The recovering step may be further comprised after the culturing step.

The recovering may be collecting the desired L-amino acid using the method of culturing the microorganism of the present disclosure, for example, using a suitable method known in the art according to a batch culture, continuous culture, or fed-batch culture method. For example, centrifugation, filtration, treatment with a protein crystallizing precipitant (salting-out method), extraction, sonication, ultrafiltration, dialysis, various kinds of chromatographies such as molecular sieve chromatography (gel filtration), adsorption chromatography, ion exchange chromatography, affinity chromatography, etc., HPLC or a combination thereof may be used, and the desired substance, specifically the L-amino acid, may be recovered from the medium or the microorganisms using suitable methods known in the art.

Further, the method of producing an L-amino acid of the present disclosure may further comprise a purification step, which may be performed using an appropriate method known in the art. In one embodiment, when the method of producing an L-amino acid of the present disclosure comprises both a recovering step and a purification step, the recovering step and the purification step may be performed continuously or intermittently regardless of the order, or simultaneously, or may be integrated into one step, but the method is not limited thereto.

In the method of the present disclosure, the NAD-dependent isocitrate dehydrogenase, introduction, and L-amino acid, etc. are as described in other aspects above.

Still another aspect of the present disclosure provides a composition for producing an L-amino acid, the composition comprising the microorganism of the genus *Corynebacterium* having an ability to produce an L-amino acid, into which the NAD-dependent isocitrate dehydrogenase protein derived from *Streptococcus mutans* of the present disclosure or the polynucleotide encoding the same is introduced, a culture of the microorganism, a fermentation product of the microorganism, or a combination of two or more thereof.

The composition of the present disclosure may further comprise any suitable excipient commonly used in compositions for producing an L-amino acid, and such excipients may comprise, for example, preservatives, wetting agents, dispersing agents, suspending agents, buffers, stabilizers, or isotonic agents, etc., but are not limited thereto.

In one specific embodiment, each component present in the composition of the present disclosure may be comprised in a microbiologically effective amount, or in an amount that may be appropriately present in the composition for production.

In the composition of the present disclosure, the NAD-dependent isocitrate dehydrogenase, introduction, and L-amino acid, etc. are as described in other aspects above.

Still another aspect of the present disclosure provides use of the microorganism of the genus *Corynebacterium* having an ability to produce an L-amino acid, into which the NAD-dependent isocitrate dehydrogenase protein derived from *Streptococcus mutans* of the present disclosure or the polynucleotide encoding the same is introduced, in producing the L-amino acid.

In the use of the present disclosure, the NAD-dependent isocitrate dehydrogenase, introduction, and L-amino acid, etc. are as described in other aspects above.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Exploration and Selection of NAD-Dependent Isocitrate Dehydrogenase (icd) Gene

In order to select organisms possessing NAD-dependent isocitrate dehydrogenase (icd), a BLAST search was performed using the amino acid sequence of endogenous isocitrate dehydrogenase of *Corynebacterium glutamicum* as a query sequence, based on the NCBI and Kegg databases. As a result, three types of microorganisms expected to possess NAD-dependent isocitrate dehydrogenase were selected, as shown in Table 1 below, in consideration of biosafety levels, which are applicable to the production strains, and availability.

**[Table 1]**

| Order | Strain | Protein Registration No. | Biosafety level |
|---|---|---|---|
| 1 | *Streptococcus mutans* | WP_002276545.1 | 1 |
| 2 | *Streptococcus* sp. *'caviae'* | WP_075346121.1 | 1 |
| 3 | *Streptococcus varani* | WP_093650533.1 | 1 |

### Example 2. Preparation of L-Tryptophan-Producing Microorganisms Introduced with Foreign NAD-Dependent Isocitrate Dehydrogenase

### Example 2-1. Preparation of Plasmid for Replacing Genes

To replace the *icd* gene in the chromosome of *Corynebacterium glutamicum,* a plasmid pDC24 (SEQ ID NO: 86) was used as a parent vector to construct a plasmid capable of replacing a coding sequence of the endogenous *icd* gene with a coding sequence of a foreign *icd* gene.

In detail, chromosomal DNA of *Corynebacterium glutamicum* ATCC13869 was used as a template, a primer pair of SEQ ID NO: 7 and SEQ ID NO: 8 was used to amplify the upstream region where homologous recombination on the chromosome occurs, and a primer pair of SEQ ID NO: 9 and SEQ ID NO: 10 was used to amplify the downstream region, and then PCR was performed to obtain respective gene fragments.

PCR was performed using Solg^{™} Pfu-X DNA polymerase (SolGent Co.), and the amplification was performed under conditions of denaturation at 95°C for 4 minutes, followed by 27 cycles of denaturation at 95°C for 30 seconds, annealing at 60°C for 30 seconds, and polymerization at 72°C for 50 seconds, and then polymerization at 72°C for 5 minutes. The primer sequences used are shown in Table 2 below.

**[Table 2]**

| SEQ ID NO. | Sequence (5'→ 3') |
|---|---|
| SEQ ID NO: 7 | TTCTATTGACTTATTTATAGAGTCTCTTCACAAA AAGCGC |
| SEQ ID NO: 8 | TCGACTCTAGAGGATCCCCGGTTTGGGATTCG TTGTAATC |
| SEQ ID NO: 9 | AATTCGAGCTCGGTACCCTGCTGAATATATGGG TGG |
| SEQ ID NO: 10 | CTTACTTTTTCTGCCATGAGTCTCCTTGGTTGA TGG |

The upstream fragment and downstream fragment of the region where homologous recombination on the chromosome occurs, amplified through the above PCR, and a chromosomal integration vector pDC24 digested with smal restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDC24△icd(13869).

### Example 2-2. Preparation of Tryptophan-Producing Microorganism of Genus Corynebacterium Introduced with NAD-Dependent Isocitrate Dehydrogenase Derived from Streptococcus mutans

The NAD-dependent isocitrate dehydrogenase derived from *Streptococcus mutans* selected in Example 1 has an amino acid sequence of SEQ ID NO: 1. Information on the gene encoding the NAD-dependent isocitrate dehydrogenase and the surrounding base sequence was obtained from the US NIH GenBank, and based on this, a codon-optimized *icd* gene derived from *Streptococcus mutans* (SEQ ID NO: 2) was synthesized using the gene synthesis service of Bionics Co., Ltd. To insert the gene into the genomic DNA of *Corynebacterium glutamicum,* the synthesized gene was used as a template and amplified by PCR using a primer pair of SEQ ID NO: 11 and SEQ ID NO: 12. Solg^{™} Pfu-X DNA polymerase was used as a polymerase, and PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes. The primer sequences used are shown in Table 3 below.

**[Table 3]**

| SEQ ID NO. | Sequence (5'→ 3') |
|---|---|
| SEQ ID NO: 11 | CCCATCAACCAAGGAGACTCATGGCAGAAAAAGT AAGTTTTG |
| SEQ ID NO: 12 | AGCGCTTTTTGTGAAGAGACTCTATAAATAAGTCA ATAGAACTTC |

Subsequently, the amplified gene fragment of NAD-dependent isocitrate dehydrogenase derived from *Streptococcus mutans,* and the chromosomal integration vector pDC24△icd(13869) digested with smal restriction enzyme, which was prepared in Example 2-1, were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDC24△icd(13869)-icd(S.mu). The cloning was performed by mixing the Gibson assembly reagent, and the gene fragment and the digested vector in appropriate molar ratios, followed by incubating at 50°C for 1 hour. The pDC24△icd(13869)-icd(S.mu) vector thus prepared was transformed into the CM05-9157 strain (US Patent Publication No. US 2023-0134555 A1), which is a tryptophan-producing strain, by electroporation (Appl. Microbiol. Biotechnol. (1999) 52:541-545) and then subjected to a double crossover to obtain a strain, in which the coding sequence of the endogenous *icd* gene on the chromosome was replaced with the coding sequence of the *icd* gene derived from *Streptococcus mutans.* The resulting strain was identified through PCR using primers of SEQ ID NO: 13 and SEQ ID NO: 14, which may respectively amplify the external regions of the homologous recombination upstream region and downstream region of the position where the gene was replaced, and genome sequencing. The primer sequences used are shown in Table 4 below.

**[Table 4]**

| SEQ ID NO. | Sequence (5'→ 3') |
|---|---|
| SEQ ID NO: 13 | CATTCGTCGATCTCTAGT |
| SEQ ID NO: 14 | CAATTCCTTGTCGGTGTC |

The strain obtained by the above method was named CM05-9157 △ icd::icd(S.mu).

### Example 2-3. Preparation of Tryptophan-Producing Microorganism of Genus Corynebacterium Introduced with NAD-Dependent Isocitrate Dehydrogenase Derived from Streptococcus sp. 'caviae'

The NAD-dependent isocitrate dehydrogenase derived from *Streptococcus* sp. '*caviae*' selected in Example 1 has an amino acid sequence of SEQ ID NO: 3. Information on the corresponding gene and the surrounding base sequence was obtained from the US NIH GenBank, and based on this, a codon-optimized icd gene derived from *Streptococcus* sp. *'caviae'* (SEQ ID NO: 4) was synthesized using the gene synthesis service of Bionics Co., Ltd. To insert the gene into the genomic DNA of *Corynebacterium glutamicum,* the synthesized gene was used as a template and amplified by PCR using a primer pair of SEQ ID NO: 15 and SEQ ID NO: 16 in the same manner as in Example 2-2. The primer sequences used are shown in Table 5 below.

**[Table 5]**

| SEQ ID NO. | Sequence (5'→ 3') |
|---|---|
| SEQ ID NO: 15 | CCCATCAACCAAGGAGACTCatggcggaaaaaattcg ctttg |
| SEQ ID NO: 16 | AGCGCTTTTTGTGAAGAGACTcaggttatccgccagc actttc |

Subsequently, the amplified gene fragment of NAD-dependent isocitrate dehydrogenase derived from *Streptococcus* sp. *'caviae',* and the chromosomal integration vector pDC24△icd(13869) digested with smal restriction enzyme, which was prepared in Example 2-1, were cloned using the Gibson assembly method to obtain a recombinant plasmid, which was named pDC24△icd(13869)-icd(S.ca). The cloning was performed by mixing the Gibson assembly reagent, the gene fragment, and the digested vector in appropriate molar ratios, followed by incubation at 50°C for 1 hour. The pDC24△icd(13869)-icd(S.ca) vector thus prepared was transformed into the CM05-9157 strain, which is a tryptophan-producing strain, by electroporation and then subjected to a double crossover to obtain a strain, in which the coding sequence of the endogenous *icd* gene on the chromosome was replaced with the coding sequence of the *icd* gene derived from *Streptococcus* sp. *'caviae'.* The resulting strain was identified through PCR using primers of SEQ ID NO: 13 and SEQ ID NO: 14 of Table 4, which may respectively amplify the external regions of the homologous recombination upstream region and downstream region of the position where the gene was replaced, and genome sequencing.

The strain obtained by the above method was named CM05-9157 △ icd::icd(S.ca).

### Example 2-4. Preparation of Tryptophan-Producing Microorganism of Genus Corynebacterium Introduced with NAD-Dependent Isocitrate Dehydrogenase Derived from Streptococcus varani

The NAD-dependent isocitrate dehydrogenase derived from *Streptococcus varani* selected in Example 1 has an amino acid sequence of SEQ ID NO: 5. Information on the corresponding gene and the surrounding base sequence was obtained from the US NIH GenBank, and based on this, a codon-optimized icd gene derived from *Streptococcus varani* (SEQ ID NO: 6) was synthesized using the gene synthesis service of Bionics Co., Ltd. To insert the gene into the genomic DNA of *Corynebacterium glutamicum,* the synthesized gene was used as a template and amplified by PCR using a primer pair of SEQ ID NO: 17 and SEQ ID NO: 18 in the same manner as in Example 2-2. The primer sequences used are shown in Table 6 below.

**[Table 6]**

| SEQ ID NO. | Sequence (5'→ 3') |
|---|---|
| SEQ ID NO: 17 | CCCATCAACCAAGGAGACTCatggcggaaaaaattgtg c |
| SEQ ID NO: 18 | AGCGCTTTTTGTGAAGAGACTgcgttcgccaatgctatc aatc |

Subsequently, the amplified gene fragment of NAD-dependent isocitrate dehydrogenase derived from *Streptococcus varani,* and the chromosomal integration vector pDC24△icd(13869) digested with smal restriction enzyme, which was prepared in Example 2-1, were cloned using the Gibson assembly method to obtain a recombinant plasmid, which was named pDC24△icd(13869)-icd(S.va). The cloning was performed by mixing the Gibson assembly reagent, the gene fragment, and the digested vector in appropriate molar ratios, followed by incubation at 50°C for 1 hour. The pDC24△icd(13869)-icd(S.va) vector thus prepared was transformed into the CM05-9157 strain, which is a tryptophan-producing strain, by electroporation and then subjected to a double crossover to obtain a strain, in which the coding sequence of the endogenous *icd* gene on the chromosome was replaced with the coding sequence of the *icd* gene derived from *Streptococcus varani.* The resulting strain was identified through PCR using primers of SEQ ID NO: 13 and SEQ ID NO: 14, which may respectively amplify the external regions of the homologous recombination upstream region and downstream region of the position where the gene was replaced, and genome sequencing.

The strain obtained by the above method was named CM05-9157 △ icd::icd(S.va).

### Example 3. Evaluation of L-Tryptophan-Producing Ability of Tryptophan-Producing Microorganisms of Genus Corynebacterium Introduced with Foreign NAD-Dependent Isocitrate Dehydrogenase Gene

In order to examine the L-tryptophan-producing ability of the CM05-9157 △ icd::icd(S.mu), CM05-9157 △ icd::icd(S.ca), CM05-9157 △ icd::icd(S.va) strains prepared in Examples 2-2, 2-3, and 2-4, respectively, and the parent strain CM05-9157, into which a foreign gene was not introduced, the strains were cultured in the following manner.

Each of the strains was inoculated into a 250-mL corner-baffled flask comprising 25 mL of a seed medium, and cultured with shaking at 200 rpm at 30°C for 20 hours. Next, 1 mL of the seed culture solution was inoculated into a 250-mL corner-baffled flask comprising 25 mL of a production medium, and cultured with shaking at 200 rpm at 30°C for 24 hours. After completing the culture, the production amount of L-tryptophan was measured by HPLC. The compositions of the seed medium and production medium are as follows, and the L-tryptophan concentration in the culture medium of each strain tested is shown in Table 7 below.

### <Seed Medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄ 7H₂O, 100 µg of biotin, 1000 µg of thiamine HCl, 2000 µg of calcium-pantothenate, 2000 µg of nicotinamide (based on 1 liter of distilled water).

### <Production Medium (pH 7.0)>

30 g of glucose, 15 g of (NH₄)₂SO₄, 1.2 g of MgSO₄ 7H₂O, 1 g of KH₂PO₄, 5 g of yeast extract, 900 µg of biotin, 4500 µg of thiamine HCl, 4500 µg of calcium-pantothenate, 30 g of CaCO₃ (based on 1 liter of distilled water).

**[Table 7]**

| | Production amount of tryptophan (g/L) | Yield of tryptophan (%) |
|---|---|---|
| CM05-9157 | 1.83 | 6.27 |
| CM05-9157 △ icd::icd(S.mu) | 2.32 | 7.94 |
| CM05-9157 △ icd::icd(S.ca) | 1.77 | 6.05 |
| CM05-9157 △ icd::icd(S.va) | 1.82 | 6.21 |

As a result, as shown in Table 7, the CM05-9157 △ icd::icd(S.mu) strain introduced with the gene derived from *Streptococcus mutans* produced final 2.32 g/L of L-tryptophan in the flask culture, confirming that the fermentation yield was increased by about 26.6%, as compared to that of the parent strain CM05-9157 which produced 1.83g/L. In contrast, the CM05-9157 △ icd::icd(S.ca) and CM05-9157 △ icd::icd(S.va) strains, each introduced with the gene derived from *Streptococcus* sp. *'caviae'* and the gene derived from *Streptococcus varani,* produced 1.77 g/L and 1.82 g/L of L-tryptophan, respectively, confirming that the yield was decreased by about 3.4% and 0.9%, respectively, as compared to that of the parent strain CM05-9157.

These results imply that the L-tryptophan-producing ability may be greatly increased specifically only when the *Streptococcus mutans-*derived NAD-dependent isocitrate dehydrogenase gene, among the foreign NAD-dependent isocitrate dehydrogenases, was introduced into the microorganism of the genus *Corynebacterium.*

### Example 4. Preparation of L-Histidine-Producing Microorganisms Introduced with Foreign NAD-Dependent Isocitrate Dehydrogenase

### Example 4-1. Preparation of Histidine-Producing Microorganism of Genus Corynebacterium

To evaluate L-histidine producing ability, a wild-type *Corynebacterium glutamicum* ATCC13032 was used as a starting microorganism to prepare the CA14-0114 strain, in which genes in the histidine biosynthetic pathway were enhanced.

In detail, to release feedback inhibition of the HisG protein which is the first enzyme in the L-histidine biosynthetic pathway, the codon of the *hisG* gene was modified to produce a protein (SEQ ID NO: 19) in which amino acids at positions 233 and 235 from the N-terminus of HisG were simultaneously substituted from glycine to histidine and from threonine to glutamine, respectively (ACS Synth. Biol., 2014, 3 (1), pp 21-29). Further, to enhance the activity of the *hisE* gene, which is present within the same operon as *hisG*, the start codon was substituted from GTG to ATG. Additionally, to enhance the L-histidine biosynthetic pathway, the promoters of the biosynthetic genes *hisN*, *hisH*, *hisD*, *hisA*, and *hisB* were replaced with strong promoters, and the pathway was enhanced by introducing additional copies of the *hisE(g1a)G(G233H*/*T235Q)* operon and *hisD* gene.

### Example 4-1-1. Preparation of Histidine-Producing Microorganism with Feedback Inhibition Released

To prepare a histidine-producing strain, in which feedback inhibition was released, PCR was performed using the chromosomal DNA of *Corynebacterium glutamicum* ATCC13032 as a template and primer pairs of SEQ ID NO: 20 and SEQ ID NO: 21, and SEQ ID NO: 22 and SEQ ID NO: 23. PCR was performed using the two amplified DNA fragments as templates and the primers of SEQ ID NO: 20 and SEQ ID NO: 23 in the same manner as in Example 2 to obtain a *'hisE(g1a)G(G233H*/*T235Q)'* gene fragment. In addition, PCR was performed using the chromosomal DNA of ATCC13032 as a template and primers of SEQ ID NOS: 71 and 72 to obtain the upstream region of the *hisE* gene.

**[Table 8]**

| SEQ ID NO. | Sequence (5'→3') |
|---|---|
| SEQ ID NO: 20 | gaggagatcaaaacaGTGAAGACATTTGAC |
| SEQ ID NO: 21 | ACACCAAGCTTGATGGATACCTCTACTGCA |
| SEQ ID NO: 22 | CAGTAGAGGTATCCATCAAGCTTGGTGTC |
| SEQ ID NO: 23 | ACTCTAGAGGATCCCCCTAGATGCGGGC |
| SEQ ID NO: 71 | TCGAGCTCGGTACCCACCGAACTCCTGACAGAGT |
| SEQ ID NO: 72 | acatgaagcgccTCGGTACATTCTTCCACA |
| SEQ ID NO: 25 | AAGAATGTACCGAggcgcttcatgtcaaca |
| SEQ ID NO: 26 | CAAATGTCTTCATtgttttgatctcctcca |
| SEQ ID NO: 27 | ACTGCCTGGTACCACCAGA |
| SEQ ID NO: 28 | CTGCCTCTCACAAGTTGAAG |

To replace with a strong promoter, PCR was performed using a synthetic promoter Pspl13 promoter (SEQ ID NO: 24, Korean Patent No. 10-1783170) as a template and primers of SEQ ID NO: 25 and SEQ ID NO: 26 in the same manner as in Example 1. After treating a pDC24 vector with restriction enzyme Sma1, the amplified upstream DNA fragment of the *hisE* gene, Pspl13 promoter, and *'hisE(g1a)G(G233H*/*T235Q)'* gene fragment were cloned using the Gibson assembly method to obtain a recombinant plasmid, which was named pDC24△Pn_hisEG:: Pspl13_hisE(g1a)G(G233H/T235Q). Gibson cloning was performed in the same manner as in Example 1.

The constructed pDC24△Pn_hisEG:: Pspl13_hisE(g1a)G(G233H/T235Q) vector was transformed into *Corynebacterium glutamicum* ATCC13032 by electroporation, and then subjected to a second crossover process to obtain a strain, in which feedback inhibition was released by introducing mutations into the existing *hisG* gene, and *hisE* activity was enhanced by replacing the start codon of the *hisE* gene. The corresponding genetic modification was confirmed by PCR using primers of SEQ ID NO: 27 and SEQ ID NO: 28 and genome sequencing. The strain thus obtained was designated as CJ-HIS1.

### Example 4-1-2. Preparation of Histidine-Producing Microorganism with Enhanced Biosynthetic Pathway through Promoter Replacement

Next, to enhance the activities of biosynthetic genes *hisN*, *hisH*, *hisD*, *hisA*, and *hisB*, plasmids for replacing the wild-type promoter of each gene with a stronger promoter were prepared.

In detail, PCR was performed using chromosomal DNA of *Corynebacterium glutamicum* ATCC13032 as a template and primers of SEQ ID NO: 29 and SEQ ID NO: 30, SEQ ID NO: 31 and SEQ ID NO: 32, SEQ ID NO: 33 and SEQ ID NO: 34, SEQ ID NO: 35 and SEQ ID NO: 36, and SEQ ID NO: 37 and SEQ ID NO: 38 in the same manner as in Example 2. The upstream regions of *hisN*, *hisH*, *hisD*, *hisA*, and *hisB* genes were obtained.

In addition, the downstream regions of the *hisN*, *hisH*, *hisD*, *hisA*, and *hisB* genes were obtained using the chromosomal DNA of *Corynebacterium glutamicum* ATCC13032 as a template and primers of SEQ ID NO: 73 and SEQ ID NO: 74, SEQ ID NO: 75 and SEQ ID NO: 76, SEQ ID NO: 77 and SEQ ID NO: 78, SEQ ID NO: 79 and SEQ ID NO: 80, and SEQ ID NO: 81 and SEQ ID NO: 82, respectively.

**[Table 9]**

| | |
|---|---|
| SEQ ID NO. | Sequence (5'→3') |
| SEQ ID NO: 29 | TCGAGCTCGGTACCCATTGGTGCTCGGCGC |
| SEQ ID NO: 30 | tgggatgtttctGTGTTGTTAGTCTAGTG |
| SEQ ID NO: 31 | TCGAGCTCGGTACCCAACCAAGTTTAGATGCGC C |
| SEQ ID NO: 32 | gctgggatgtttctGCCGATAGTTTATGTCA |
| SEQ ID NO: 33 | TCGAGCTCGGTACCCGGTGACAGCTCGCGCCG CAT |
| SEQ ID NO: 34 | gcgctgggatgtttctGGCGAAAAGTTCTCCC |
| SEQ ID NO: 35 | TCGAGCTCGGTACCCTTGATGCCTGCATGAAG G |
| SEQ ID NO: 36 | tgacatgaagcgccGAATATTGATCCTATCT |
| SEQ ID NO: 37 | TTCGAGCTCGGTACCCACCTTCAGCAACCACTC |
| SEQ ID NO: 38 | tgacatgaagcgccGAAAAATTCTTCTCT |
| SEQ ID NO: 73 | aaaggaaacactcATGAGCAAATATGCAGACG |
| SEQ ID NO: 74 | CTAGAGGATCCCCCAGCCGGAGAGGGA |
| SEQ ID NO: 75 | aaaggaaacactcATGACCAAAACTGTCGC |
| SEQ ID NO: 76 | CTAGAGGATCCCCACCTCTGGAGGCGTGGTC |
| SEQ ID NO: 77 | cgaaaggaaacactcATGTTGAATGTCACTGACC |
| SEQ ID NO: 78 | CTAGAGGATCCCCCCGTGCTCAGCCTGAGGAG |
| SEQ ID NO: 79 | ggagatcaaaacaATGACCTTCACTATTCTTCC |
| SEQ ID NO: 80 | CTAGAGGATCCCCACGAAACGTGCACAACCTT |
| SEQ ID NO: 81 | gagatcaaaacaATGACTGTCGCACCA |
| SEQ ID NO: 82 | CTAGAGGATCCCCGGGTCGCGGCCGTAGTGGC |

To replace the endogenous promoters of *hisN*, *hisH* and *hisD* genes with a strong promoter Pcj7 promoter (SEQ ID NO: 39, Patent No. 10-0620092), PCR was performed in the same manner as in Example 2 using the genomic DNA of *Corynebacterium ammoniagenes* as a template and primers of SEQ ID NO: 40 and SEQ ID NO: 41, SEQ ID NO: 42 and SEQ ID NO: 43, and SEQ ID NO: 83 and SEQ ID NO: 84.

**[Table 10]**

| SEQ ID NO. | Sequence (5'→3') |
|---|---|
| SEQ ID NO: 40 | ACTAGACTAACAACACagaaacatcccagc gc |
| SEQ ID NO: 41 | TCTGCATATTTGCTCATgagtgtttccttt |
| SEQ ID NO: 42 | ACATAAACTATCGGCagaaacatcccagcg cta |
| SEQ ID NO: 43 | GACAGTTTTGGTCATgagtgtttcctttcg |
| SEQ ID NO: 83 | GAGAACTTTTCGCCagaaacatcccagcgc t |
| SEQ ID NO: 84 | AGTGACATTCAACATgagtgtttcctttcg |

Further, to replace the endogenous promoters of *hisA* and *hisB* genes with a strong promoter Pspl13 promoter (SEQ ID NO: 24, Korean Patent No. 10-1783170), PCR was performed in the same manner as in Example 2 using the Pspl13 promoter as a template and primers of SEQ ID NO: 44 and SEQ ID NO: 45, SEQ ID NO: 46 and SEQ ID NO: 85.

**[Table 11]**

| SEQ ID NO. | Sequence (5'→3') |
|---|---|
| SEQ ID NO: 44 | AGGATCAATATTCggcgcttcatgtcaac |
| SEQ ID NO: 45 | GAATAGTGAAGGTCATtgttttgatctcct |
| SEQ ID NO: 46 | GAGAAGAATTTTTCggcqcttcatgtcaa |
| SEQ ID NO: 85 | TGGTGCGACAGTCATtgttttqatctcct |

After treating the pDC24 vector with restriction enzyme Sma1, the amplified upstream DNA fragments of *hisN, hisH,* and *hisD* genes, the Pcj7 promoter fragment, and the downstream DNA fragments of *hisN, hisH,* and *hisD* genes were each cloned using the Gibson assembly method, thereby obtaining recombinant plasmids, which were named pDC24ΔPn:: Pcj7_hisN, pDC24ΔPn:: Pcj7_hisH, pDC24ΔPn:: Pcj7_hisD, respectively. In addition, after treating the pDC24 vector with restriction enzyme Sma1, the amplified upstream DNA fragments of *hisA* and *hisB* genes, the Pspl13 promoter fragment, and the downstream DNA fragments of *hisA* and *hisB* genes were each cloned using the Gibson assembly method, thereby obtaining recombinant plasmids, which were named pDC24ΔPn:: Pspl13_hisA, pDC24ΔPn:: Pspl13_hisB, respectively. Gibson cloning was performed in the same manner as in Example 2.

The constructed pDC24△Pn::Pcj7_hisN vector was transformed into CJ-HIS1, which was constructed in Example 4-1-1, by electroporation. Through a second crossover, the promoter was replaced in the existing *hisN* gene, thereby obtaining a strain in which the corresponding gene was enhanced. The corresponding genetic modification was confirmed by PCR using primers of SEQ ID NO: 47 and SEQ ID NO: 48 and genome sequencing. The strain thus obtained was designated as CJ-HIS2.

Next, the constructed pDC24△Pn::Pcj7_hisH vector was transformed into CJ-HIS2 prepared above by electroporation. Through a second crossover, the promoter was replaced in the existing *hisH* gene, thereby obtaining a strain in which the corresponding gene was enhanced. The corresponding genetic modification was confirmed by PCR using primers of SEQ ID NO: 49 and SEQ ID NO: 50 and genome sequencing. The strain thus obtained was designated as CJ-HIS3.

Next, the constructed pDC24△Pn::Pcj7_hisD vector was transformed into CJ-HIS3 prepared above by electroporation. Through a second crossover, the promoter was replaced in the existing *hisD* gene, thereby obtaining a strain in which the corresponding gene was enhanced. The corresponding genetic modification was confirmed by PCR using primers of SEQ ID NO: 51 and SEQ ID NO: 52 and genome sequencing. The strain thus obtained was designated as CJ-HIS4.

Next, the constructed pDC24△Pn::Pspl13_hisA vector was transformed into CJ-HIS4 prepared above by electroporation. Through a second crossover, the promoter was replaced in the existing *hisA* gene, thereby obtaining a strain in which the corresponding gene was enhanced. The corresponding genetic modification was confirmed by PCR using primers of SEQ ID NO: 53 and SEQ ID NO: 54 and genome sequencing. The strain thus obtained was designated as CJ-HIS5.

Next, the constructed pDC24△Pn::Pspl13_hisB vector was transformed into CJ-HIS5 prepared above by electroporation. Through a second crossover, the promoter was replaced in the existing *hisB* gene, thereby obtaining a strain in which the corresponding gene was enhanced. The corresponding genetic modification was confirmed by PCR using primers of SEQ ID NO: 55 and SEQ ID NO: 56 and genome sequencing. The strain thus obtained was designated as CJ-HIS6.

**[Table 12]**

| SEQ ID NO. | Sequence (5'→3') |
|---|---|
| 47 | GAGCATGCATCAAAG |
| 48 | AGAAATTTGATCCTTATAA |
| 49 | TTGAGAGATGCTTATCG |
| 50 | CACTTCAGTGCGGATTCCAA |
| 51 | AGCGGGTTTAATTCAGG |
| 52 | GTGGGTAAGGGTTTTCGT |
| 53 | CACGAAAATGATCGTTTTG |
| 54 | TATGGGATTCGATGGCCA |
| 55 | TGTGGGAATCGCTGGGCAC |
| 56 | CGGTCGCCCGCATCTG |

### Example 4-1-3. Preparation of Histidine-Producing Microorganism with Enhanced Biosynthetic Pathway through Additional Gene Insertion

Next, NCgl1021, which is known as a gene encoding a transposon in *Corynebacterium glutamicum,* was used as an insertion site to additionally insert the *hisE(g1a)G(G233H*/*T235Q)* operon and *hisD* gene. In detail, to construct a vector for NCgl1021 deletion and target gene insertion, PCR was performed in the same manner as in Example 2 using the chromosome of ATCC13032 as a template and primer pairs of SEQ ID NO: 57 and SEQ ID NO: 58, SEQ ID NO: 59 and SEQ ID NO: 60 to amplify the left homology arm region of NCgl1021 and the right homology arm region of NCgl1021.

**[Table 13]**

| SEQ ID NO. | Sequence (5'→3') |
|---|---|
| SEQ ID NO: 57 | TTCGAGCTCGGTACCCATGAAGTCTACC GGC |
| SEQ ID NO: 58 | gacatgaagcgccGACATCTAATAACCGGG |
| SEQ ID NO: 59 | CCGACGAGGCCTAAGAACTCATTCCTTC TGCT |
| SEQ ID NO: 60 | CTCTAGAGGATCCCCTTAGAGTGCATTG ATC |

PCR was performed in the same manner as in Example 2 using pDC24△Pn ::Pspl13_hisE(g1a)G(G233H/T235Q), which was the vector constructed in Example 4-1-1, as a template and primers of SEQ ID NO: 61 and SEQ ID NO: 62 to obtain a *'Pspl13_hisE(g1a)G(G233H*/*T235Q)'* gene fragment. In addition, PCR was performed in the same manner as in Example 2 using pDC24△Pn ::Pcj7_hisD, which was the vector constructed in Example 4-1-2, as a template and primers of SEQ ID NO: 63 and SEQ ID NO: 64 to obtain a 'Pcj7_hisD' gene fragment.

**[Table 14]**

| SEQ ID NO. | Sequence (5'→3') |
|---|---|
| SEQ ID NO: 61 | CCGGTTATTAGATGTCqqcqcttcatqtca |
| SEQ ID NO: 62 | ggatgtttctCTAGATGCGGGCGAT |
| SEQ ID NO: 63 | GCCCGCATCTAGagaaacatcccagcgct |
| SEQ ID NO: 64 | AGAAGGAATGAGTTCTTAGGCCTCGT CGG |

After treating the pDC24 vector with restriction enzyme Sma1, the amplified left homology arm and right homology arm regions of NCgl1021, *'Pspl13_hisE(g1a)G(G233H*/*T235Q)'* and *'Pcj7_hisD'* gene fragments were cloned using the Gibson assembly method to obtain a recombinant plasmid, which was named 'pDC24△NCgI1021::Pspl13_hisE(g1a)G(G233H/T235Q)- Pcj7_hisD'. Gibson cloning was performed in the same manner as in Example 2. The constructed 'pDC24-Pspl13_hisE(g1a)G(G233H/T235Q)-Pcj7_hisD' vector was transformed into CJ-HIS6 prepared in Example 4-1-2 by electroporation, and then through a double crossover, a strain was obtained, in which the histidine biosynthetic pathway was enhanced by additional gene insertion. The corresponding genetic modification was confirmed by PCR using primers of SEQ ID NO: 65 and SEQ ID NO: 66 and genome sequencing. The strain thus obtained was designated as CA10-0114.

**[Table 15]**

| SEQ ID NO. | Sequence (5'→3') |
|---|---|
| SEQ ID NO: 65 | CTTTCAGCTTTCCCTCCCG |
| SEQ ID NO: 66 | GCTGTACTTTTAGTACA |

### Example 4-2. Preparation of Plasmid for Replacing Genes

A plasmid was constructed to introduce the NAD-dependent isocitrate dehydrogenase derived from *Streptococcus mutans* showing excellent ability to produce tryptophan in Example 3, into the chromosome of a histidine-producing *Corynebacterium glutamicum* strain. At this time, a plasmid pDC24 was used as a parent vector to construct a plasmid capable of replacing a coding sequence of the endogenous *icd* gene with a coding sequence of an exogenous *icd* gene under a natural promoter.

In detail, chromosomal DNA of *Corynebacterium glutamicum* ATCC13032 was used as a template, a primer pair of SEQ ID NO: 67 and SEQ ID NO: 68 was used to amplify the upstream region where homologous recombination on the chromosome occurs, and a primer pair of SEQ ID NO: 69 and SEQ ID NO: 70 was used to amplify the downstream region, and then PCR was performed to obtain respective gene fragments. PCR was performed using Solg^{™} Pfu-X DNA polymerase (SolGent co.), and the amplification was performed under conditions of denaturation at 95°C for 4 minutes, followed by 27 cycles of denaturation at 95°C for 30 seconds, annealing at 60°C for 30 seconds, and polymerization at 72°C for 50 seconds, and then polymerization at 72°C for 5 minutes. The primer sequences used are shown in Table 16 below.

**[Table 16]**

| SEQ ID NO. | Sequence (5'→ 3') |
|---|---|
| SEQ ID NO: 67 | GAATTCGAGCTCGGTACCCCTCATTTTTCCCAAC CCC |
| SEQ ID NO: 68 | ttcttcaaaacttactttttctqccatGAGTCTCCTTGGTTGATG |
| SEQ ID NO: 69 | ttctattgacttatttatagAGTCTCTTCACAAAAAGCGC |
| SEQ ID NO: 70 | CCTGCAGGTCGACTCTAGAGGATCCCCTTGAGTT AGTCCACGCCC |

The upstream fragment and downstream fragment of the region where homologous recombination on the chromosome occurs, amplified through the above PCR, and a chromosomal integration vector pDC24 digested with smal restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDC24△icd(13032).

### Example 4-3. Preparation of Histidine-Producing Microorganism of Genus Corynebacterium Introduced with NAD-Dependent Isocitrate Dehydrogenase Derived from Streptococcus mutans

To introduce the gene encoding the NAD-dependent isocitrate dehydrogenase (SEQ ID NO: 1) derived from *Streptococcus mutans* which was selected in Example 1 into the histidine-producing *Corynebacterium glutamicum* strain, PCR was performed using the *icd* gene of *S*. *mutans* (SEQ ID NO: 2) as a template and primers of SEQ ID NO: 11 and SEQ ID NO: 12 of Table 3 as described in Example 2-2. Solg^{™} Pfu-X DNA polymerase was used as a polymerase, and PCR was performed under PCR amplification conditions of denaturation at 95°C for 2 minutes, followed by 27 cycles of denaturation at 95°C for 20 seconds, annealing at 60°C for 40 seconds, and polymerization at 72°C for 1 minute, and then polymerization at 72°C for 5 minutes.

Subsequently, the amplified *Streptococcus mutans*-derived NAD-dependent isocitrate dehydrogenase gene fragment and the chromosomal integration vector pDC24△icd(13032) constructed in Example 2-1, which was digested with Smal restriction enzyme, were cloned using the Gibson assembly method (DG Gibson et al., NATURE METHODS, VOL.6 NO.5, MAY 2009, NEBuilder HiFi DNA Assembly Master Mix) to obtain a recombinant plasmid, which was named pDC24△icd(13032)-icd(S.mu). The cloning was performed by mixing the Gibson assembly reagent, the gene fragment, and the digested vector in appropriate molar ratios, followed by incubation at 50°C for 1 hour.

The pDC24△icd(13032)-icd(S.mu) vector thus prepared was transformed into the histidine-producing strain CA14-0114, which was prepared in Example 4-1, by electroporation (Appl. Microbiol.Biotechnol. (1999) 52:541-545) and then subjected to a double crossover to obtain a strain, in which the coding sequence of the endogenous *icd* gene on the chromosome was replaced with the coding sequence of the *icd* gene derived from *Streptococcus mutans.* The resulting strain was identified through PCR using primers of SEQ ID NO: 13 and SEQ ID NO: 14 of Table 4, which may respectively amplify the external regions of the homologous recombination upstream region and downstream region of the position where the gene was replaced, and genome sequencing.

The strain obtained by the above method was named CA14-0114△icd::icd(S.mu).

### Example 5. Evaluation of L-Histidine-Producing Ability of Microorganisms of Genus Corynebacterium Introduced with Foreign NAD-Dependent Isocitrate Dehydrogenase Gene

In order to examine the L-histidine-producing ability of the CA14-0114 △ icd::icd (S.mu) strain prepared in Example 4, and the parent strain CA14-0114, into which a foreign gene was not introduced, the strains were cultured in the following manner.

Each of the strains was inoculated into a 250-mL corner-baffled flask comprising 25 mL of a seed medium, and cultured with shaking at 200 rpm at 30°C for 48 hours. Next, 1 mL of the seed culture solution was inoculated into a 250-mL corner-baffled flask comprising 25 mL of a production medium, and cultured with shaking at 200 rpm at 30°C for 24 hours. After completing the culture, the production amount of L-histidine was measured by HPLC. The compositions of the seed medium and production medium are as follows, and the L-histidine concentration in the culture medium for each strain tested is shown in Table 17 below.

### <Seed Medium (pH 7.0)>

5% glucose, 1% bacto-peptone, 0.25% sodium chloride, 1% yeast extract, 0.4% urea (based on 1 liter of distilled water)

### <Production Medium (pH 7.0)>

6% raw sugar, 2% ammonium sulfate, 0.1% monobasic potassium phosphate, 0.05% magnesium sulfate heptahydrate, 2.0% corn steep liquor (CSL), 200 µg/L biotin, 30 g/L calcium carbonate (based on 1 liter of distilled water)

**[Table 17]**

| | Production amount of histidine (g/L) | Production amount of histidine (%) |
|---|---|---|
| CA14-0114 | 4.6 | 100 |
| CA14-0114 △ icd::icd(S.mu) | 6.2 | 134.8 |

As a result, as shown in Table 17, the CA14-0114△icd::icd(S.mu) strain, which was introduced with the NAD-dependent isocitrate dehydrogenase gene derived from *Streptococcus mutans,* produced final 6.2 g/L of L-histidine in the flask culture, confirming that the fermentation yield was increased by about 34.8%, as compared to that of the parent strain CA14-0114 which produced 4.6 g/L.

These results imply that the introduction of the *Streptococcus mutans-*derived NAD-dependent isocitrate dehydrogenase gene into the microorganism of the genus *Corynebacterium* not only increases the L-tryptophan-producing ability as confirmed in Example 3, but also increases the L-histidine-producing ability.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A microorganism of the genus *Corynebacterium* having an ability to produce an L-amino acid, into which an NAD-dependent isocitrate dehydrogenase protein derived from *Streptococcus mutans* or a polynucleotide encoding the same is introduced.

2. The microorganism of the genus *Corynebacterium* of claim 1, wherein the L-amino acid is any one or more selected from the group consisting of L-tryptophan and L-histidine.

3. The microorganism of the genus *Corynebacterium* of claim 1, wherein the NAD-dependent isocitrate dehydrogenase protein derived from *Streptococcus mutans* comprises an amino acid sequence of SEQ ID NO: 1 or an amino acid sequence having 80% or more sequence identity thereto.

4. The microorganism of the genus *Corynebacterium* of claim 1, wherein the NAD-dependent isocitrate dehydrogenase protein derived from *Streptococcus mutans* is encoded by *icd* gene.

5. The microorganism of the genus *Corynebacterium* of claim 1, wherein the polynucleotide encoding the NAD-dependent isocitrate dehydrogenase protein derived from *Streptococcus mutans* comprises a nucleotide sequence of SEQ ID NO: 2.

6. The microorganism of the genus *Corynebacterium* of claim 1, wherein the microorganism of the genus *Corynebacterium* is *Corynebacterium glutamicum.*

7. The microorganism of the genus *Corynebacterium* of any one of claims 1 to 6, wherein the microorganism of the genus *Corynebacterium* has an increased ability to produce an L-amino acid, as compared to an unmodified microorganism.

8. A method of producing an L-amino acid, the method comprising the step of culturing, in a medium, a microorganism of the genus *Corynebacterium* having an ability to produce an L-amino acid, into which an NAD-dependent isocitrate dehydrogenase protein derived from *Streptococcus mutans* or a polynucleotide encoding the same is introduced.

9. The method of claim 8, wherein the L-amino acid is any one or more selected from the group consisting of L-tryptophan and L-histidine.

10. The method of claim 8, further comprising the step of recovering the L-amino acid from the cultured microorganism, a culture of the microorganism, a fermentation product of the microorganism, or the culture medium.

11. A composition for producing an L-amino acid, the composition comprising a microorganism of the genus *Corynebacterium* having an ability to produce an L-amino acid, into which an NAD-dependent isocitrate dehydrogenase protein derived from *Streptococcus mutans* or a polynucleotide encoding the same is introduced, a culture of the microorganism, a fermentation product of the microorganism, or a combination of two or more thereof.

12. The composition of claim 11, wherein the L-amino acid is any one or more selected from the group consisting of L-tryptophan and L-histidine.

13. Use of a microorganism of the genus *Corynebacterium* having an ability to produce an L-amino acid, into which an NAD-dependent isocitrate dehydrogenase protein derived from *Streptococcus mutans* or a polynucleotide encoding the same is introduced, in producing the L-amino acid.
